# EUROPEAN PATENT APPLICATION

(11) **EP 4 365 910 A1**
(43) Date of publication of application: **08.05.2024**
(21) Application number: 22205852.1
(22) Date of filing: 07.11.2022
(51) Int. Cl.: G16H 40/20, G16H 30/40, G06N 3/08, G06N 20/00, G06V 10/764, G06V 10/82, G05B 23/02

(54) **SYSTEM AND METHOD FOR MONITORING A CLEANING PROCESS IN A MEDICAL ENVIRONMENT**

(71) Applicant: Koninklijke Philips N.V., 5656 AG Eindhoven (NL)
(72) Inventor: KRUEGER, Sascha, 5656AG Eindhoven (NL); DUENSING, George Randall, 5656AG Eindhoven (NL)
(74) Representative: Philips Intellectual Property & Standards

(57) **Abstract**

In a medical environment (1) a cleaning process (2) for cleaning of surfaces (4) is to be improved. This is achieved by providing a system for monitoring a cleaning process (2) of a surface (4) in a medical environment (1). The system comprises at least one camera unit (8) for taking digital images of the medical environment (1), with at least one sensor for a 3D detection of the medical environment (1), at least one memory (11) storing instructions; and at least one processor (10) that executes the instructions. The processor is configured in such a way to cause the following to be performed: detecting a cleaning device (5) for applying a cleaning agent (7) to the surface in the medical environment (1) from a digital image taken by the camera unit (8) and/or detecting of at least one body part of a person (14) in the medical environment (1) from the digital image taken by the camera unit (8), tracking the cleaning device (5) along a cleaning trajectory (6) in the medical environment (1) and/or detecting at least one body part of the person (14) and tracking the body part of the person (14) in the medical environment (1), deriving a contact probability of the cleaning device (1) with the surface (4) from the cleaning trajectory (6) and/or deriving a contact probability of the at least one body part of the person (14) with the surface (4). The present invention also concerns a computer-implemented method for monitoring a cleaning process of a surface (4) in a medical environment (1). A computer-implemented method for training an artificial intelligence algorithm to evaluate data.

## Description

### FIELD OF THE INVENTION

The invention relates to the field of cleaning of a medical environment, and in particular to a system and a method for monitoring a cleaning process of surfaces and objects in a medical environment.

### BACKGROUND OF THE INVENTION

Disinfection of a medical environment is a time-consuming task. Controlling the quality of the cleaning result on a daily basis is also challenging. To address this issue, disinfection robots are being developed. However, many environments do not allow the use of robots e.g., due to space constraints or compatibility with the medical equipment. On top cleaning is a 6D task involving a multitude of spatial scales e.g., large floor areas vs. tiny grooves and notches. Such tasks are yet too complex and thus prohibitive for the use of robots across various industries. As a result, with today's cleaning robots, still human action would be required to achieve the final disinfection level. Similar issues apply to UV disinfection trolley systems. Additionally, UV based disinfection systems are associated with additional protective safety measures to allow other workers in the same area thus negatively impacting the overall hospital workflows.

From the document CN111738681A a disinfection behavior intelligent judgment system, a method based on deep learning and an intelligent socket for hotel disinfection work are known. The behavior judgment basis of the invention comprises two aspects of data: on the one hand, working data of cleaning personnel in a disinfection room acquired by an image recognition technology based on deep learning; on the other hand, working data of a disinfection cabinet reported by an intelligent socket. An application server performs fusion judgment on the two data by using a data fusion technology to obtain a judgment result of whether the disinfection room performs normal disinfection on that day, so the accuracy and standardization of the result are ensured. Finally, the daily disinfection work completion condition of each hotel is displayed in a centralized manner through an application platform. Remote intelligent supervision of the hotel can be realized, the cup cleaning condition of the hotel can be accurately mastered, and the supervision efficiency and the service quality are improved. However, this document concerns only a neighboring field. The requirements for cleaning medical equipment and rooms are many times higher than the requirements for cleaning hotel rooms. Compliance with the relevant standards is important, especially in the medical field. Therefore, there is a need to improve the existing procedures for cleaning and disinfecting of surfaces of medical equipment.

### SUMMARY OF THE INVENTION

It is an object of the invention to further improve the cleaning and disinfection of surfaces in a medical environment. The methods known in the state of the art are too complex or originate from a neighboring area and do not meet the requirements for the cleaning and disinfection of surfaces in a medical environment.

According to the invention, this object is addressed by the subject matter of the independent claims. Preferred embodiments of the invention are described in the sub claims.

Therefore, according to the invention, a system for monitoring a cleaning process and/or a contamination of a surface in a medical environment is provided the system comprising:
- at least one camera unit for taking digital images of the medical environment, with at least one sensor for a 3D detection of the medical environment,
- at least one memory storing instructions; and
- at least one processor that executes the instructions to cause the following to be performed:
- taking a digital image of the medical environment by the camera unit,
- detecting a cleaning device for applying a cleaning agent to the surface in the medical environment from the digital image taken by the camera unit and/or detecting of at least one body part of a person in the medical environment from the digital image taken by the camera unit,
- tracking the cleaning device along a cleaning trajectory in the medical environment and/or tracking the body part of the person in the medical environment,
- deriving a contact probability of the cleaning device with the surface from the cleaning trajectory and/or deriving a contact probability of the at least one body part of the person with the surface.

The basic idea of the present invention is to use digital images taken by the camera unit to detect and track the cleaning device along a cleaning trajectory to determine the contact probability of the cleaning device with a surface in a medical environment. The system is further set up to detect at least one body part of a person and tracking the body part of the person in the medical environment to deriving a contact probability of the at least one body part of the person with the surface. From the contact probability of the at least one body part of the person with the surface possible contamination of the surface can be concluded. Subsequent cleaning can then pay particular attention to areas that have experienced contamination. The term surface is understood to include not only surfaces such as the floor of a medical environment, but also the surfaces of objects such as medical equipment. This can be used to infer a degree of cleaning of the surface based on the contact probability of the cleaning device with the surface. The term cleaning is also understood to mean, in particular, the disinfection of surfaces in a medical environment. The cleaning device and its trajectory are thereby recognized in images captured by a camera unit. Here, the system includes a processor that performs the steps necessary to detect and track the cleaning device and/or the body part of the person.

In an advantageous embodiment of the invention the at least one processor is arranged to execute an artificial intelligence algorithm, the artificial intelligence algorithm being trained to detect the cleaning device, to track the cleaning device along a cleaning trajectory and to derive a contact probability of the cleaning device with the surface from the cleaning trajectory and/or the artificial intelligence algorithm being trained to detect at least one body part of a person in the medical environment from the digital image taken by the camera unit, to track the body part of a person and to derive a contact probability of the body part of a person with the surface.

Pattern recognition is a common feature in artificial intelligence and machine learning applications. In general, it is used to assign a label or classification to a given input value by performing "most likely" matching of the inputs, taking into account their statistical variation. Therefore, artificial intelligence algorithms can be used to detect and track objects such as cleaning devices or body parts of a person.

In an advantageous embodiment of the invention the artificial intelligence algorithm is a trained neural network that machine learns from data of known cleanings device to detect the cleaning device and to track the cleaning trajectory of the cleaning device and/or the trained neural network machine learns from data of known body parts of a person to detect the body part of a person and to track the body part of a person. In machine learning one develops and studies methods that give computers the ability to solve problems by learning from experiences. The goal is to create mathematical models that can be trained to produce useful outputs when fed input data. Machine learning models are provided experiences in the form of training data and are tuned to produce accurate predictions for the training data by an optimization algorithm. The main goal of the models is to be able to generalize their learned expertise, and deliver correct predictions for new, unseen data. A model's generalization ability is typically estimated during training using a separate data set, the validation set, and used as feedback for further tuning of the model. After several iterations of training and tuning, the final model is evaluated on a test set, used to simulate how the model will perform when faced with new, unseen data. Roughly, a neural network consists of a number of connected computational units, called neurons, arranged in layers. There's an input layer where data enters the network, followed by one or more hidden layers transforming the data as it flows through, before ending at an output layer that produces the neural network's predictions. The network is trained to output useful predictions by identifying patterns in a set of labeled training data, fed through the network while the outputs are compared with the actual labels by an objective function. During training the network's parameters - the strength of each neuron - is tuned until the patterns identified by the network result in good predictions for the training data. Once the pat- terns are learned, the network can be used to make predictions on new, unseen data, i.e., generalize to new data.

In an advantageous embodiment of the invention the camera unit includes at least one of the following sensors, an RGB camera sensor, an ultrasonic sensor for ultrasonic ranging, a LIDAR-sensor, a time-of-flight sensor and/or a sensor for a stereo or triangulation ranging technique. In an advantageous embodiment of the invention the system comprises a thermal camera and/or an audio sensor. The thermal camera allows detecting surface areas where cleaning agent was applied to using the effect of the evaporation cooling.

The invention further relates to a computer-implemented method for monitoring a contamination of a surface in a medical environment comprising:
- providing a system for monitoring a contamination of a surface in a medical environment comprising:
- at least one camera unit for taking digital images of the medical environment, with at least one sensor for a 3D detection of the medical environment,
- at least one memory storing instructions; and
- at least one processor that executes the instructions,
the method comprising the following steps:
- taking a digital image of the medical environment by the camera unit,
- detecting of at least one body part of a person in the medical environment from the digital image taken by the camera unit,
- tracking the body part of the person in the medical environment,
- deriving a contact probability of the at least one body part of the person with the surface,
- determinizing a level of contamination of the surface based on the contact probability of the at least one body part of the person with the surface,
- logging the determined level of contamination,
- providing a contamination map of the medical environment based on the logged determined level of contamination.

The invention further relates to a computer-implemented method for monitoring a cleaning process of a surface in a medical environment comprising:
- providing a system for monitoring a cleaning process of a surface in a medical environment comprising:
- at least one camera unit for taking digital images of the medical environment, with at least one sensor for a 3D detection of the surface in the medical environment,
- at least one memory storing instructions; and
- at least one processor that executes the instructions,
the method comprising the following steps:
- detecting a cleaning device from a digital image taken by the camera unit, while the cleaning device is in contact with the surface in the medical environment to apply a cleaning agent to the surface,
- tracking the cleaning device along a cleaning trajectory in the medical environment,
- deriving a contact probability of the cleaning device with the surface from the cleaning trajectory,
- determinizing a level of cleaning of the surface based on the contact probability of the cleaning device with the surface,
- logging the determined level of cleaning.

In an advantageous embodiment before the computer-implemented method for monitoring a cleaning process of a surface in a medical environment is carried out, the computer-implemented method for monitoring a contamination of a surface in a medical environment is carried out and the cleaning process is carried out based on the contamination map created therein. This procedure has the advantage that the cleaning process can be planned based on the previously determined contaminations. This allows the cleaning process to be designed effectively.

In an advantageous embodiment of the invention the method steps are at least partially performed by an artificial intelligence algorithm executed by means of the at least one processor.

In an advantageous embodiment of the invention the artificial intelligence algorithm is a trained neural network that machine learns from data of known cleanings device to detect the cleaning device and to track the cleaning trajectory of the cleaning device and/or the trained neural network machine learns from data of known body parts of a person to detect the body part of a person and to track the body part of a person.

In an advantageous embodiment of the invention the system comprises at least one thermal camera and the method comprises the following additional steps:
- detecting an evaporation cooling of the cleaning agent applied to the surface by means of the thermal camera,
- deriving an amount of applied cleaning agent based on the detected evaporation cooling.

In an advantageous embodiment of the invention the system comprises at least one audio sensor and the method comprises the following additional steps:
- measuring of sounds by means of the audio sensor in the medical environment,
- associating the sound with a cleaning activity in the medical environment.

Audio sensors can be easily added to measure friction and vibration sounds associated with cleaning activities.

In an advantageous embodiment of the invention the output of different sensors is combined in an overarching artificial intelligence algorithm, wherein a machine-learning model of the overarching artificial intelligence algorithm is trained to infer the level of achieved cleaning from the information derived by the different sensors.

The invention further relates to a computer-implemented method for training an artificial intelligence algorithm to evaluate data, comprising the steps of:
- obtaining training data for an artificial intelligence algorithm, wherein the training data comprises a plurality of base training images and respective classification data for each of the base training images, and wherein the artificial intelligence algorithm is configured to receive an input image and predict classification data for the input image, wherein each image comprises data representing cleaning devices and/or body parts of a person.

The invention further relates to a computer program product comprising instructions to cause the system as described above to execute the steps of the method as described above.

The invention further relates to a non-transitory computer readable medium storing instruction that, when executed by at least one processor, cause the method recited above to be performed.

The present general inventive concept relates to the tracking of objects in a medical environment. In principle, it does not matter what kind of objects are involved. Therefore, the tracking of cleaning devices and the tracking of body parts of a person to detect effective cleaning and a risk of contamination of a surface in the medical environment, respectively, share a general inventive concept.

### BRIEF DESCRIPTION OF THE DRAWINGS

These and other aspects of the invention will be apparent from and elucidated with reference to the embodiments described hereinafter. Such an embodiment does not necessarily represent the full scope of the invention, however, and reference is made therefore to the claims and herein for interpreting the scope of the invention.

In the drawings:
Fig. 1 schematically depicts a system for monitoring a cleaning process of a surface in a medical environment according to an embodiment of the invention,
Fig. 2 depicts a flowchart of a computer-implemented method for monitoring a cleaning process of a surface in a medical environment according to an embodiment of the invention.

### DETAILED DESCRIPTION OF EMBODIMENTS

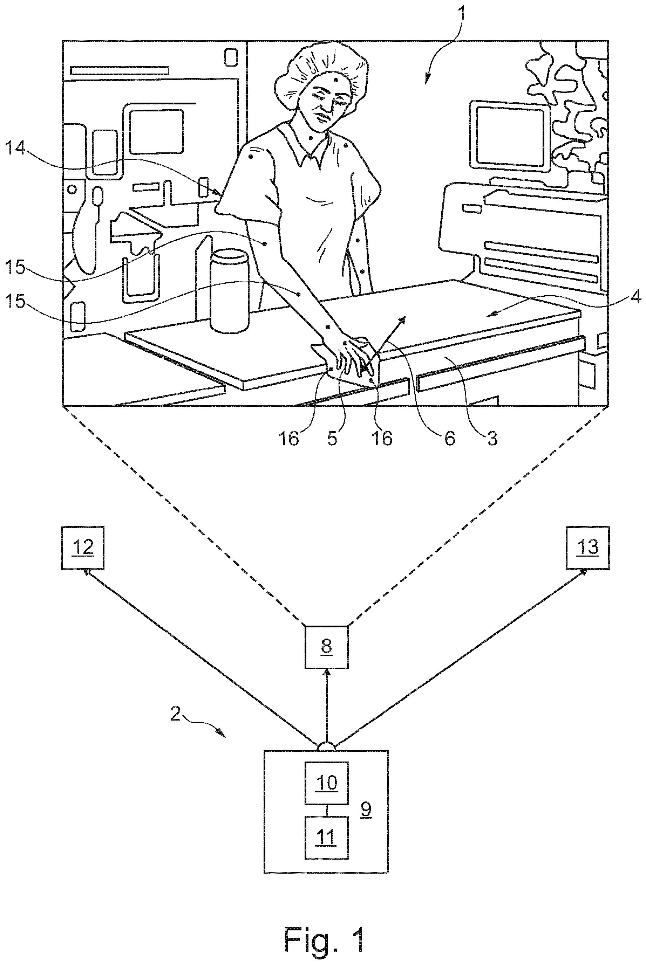

Fig. 1 schematically depicts a system for monitoring a cleaning process 2 of a surface 4 in a medical environment 1 according to an embodiment of the invention. In Fig. 1 a camera unit 8 overviewing a medical environment 1 to be cleaned is shown. Typically, the system 2 will be installed such that it can monitor the patient area, but also other areas can be equipped with the system 2. The camera unit 8 allows 3D detection of objects 3 and surfaces 4 in the medical environment 1. For this purpose, the camera unit 8 includes a at least one sensor for a 3D detection of the medical environment 1. For example, the following sensors can be considered for 3D detection of the surface 4. An RGB camera sensor, an ultrasonic sensor for ultrasonic ranging, a LIDAR-sensor, a time-of-flight sensor and/or a sensor for a stereo or triangulation ranging technique. Furthermore, the system 2 includes at least one memory 11 storing instructions and at least one processor unit 9 with at least one processor 10 that executes the instructions. By executing the instructions, at least the following steps are performed. Detecting of at least one body part of a person 14 in the medical environment 1 from the digital image taken by the camera unit 8, and tracking the body part of the person 14 in the medical environment 1. The detection and the tracking of certain body parts can in particular with the help of certain body keypoints 15 which are recorded by the sensors, e.g., the camera unit, and then evaluated by the system. As a further step it is foreseen that to derive a contact probability of the at least one body part of the person 14 with the surface 4. This allows to determinize a level of contamination of the surface 4 based on the contact probability of the at least one body part of the person 14 with the surface 4. The determined level of contamination can be logged and a contamination map of the medical environment 1 based on the logged determined level of contamination can be provided. This method can be used, for example, prior to a cleaning process in order to identify areas and surfaces 4 in the environment 1 that could be contaminated. Subsequently, a method for monitoring a cleaning process of a surface 4 in a medical environment may be provided. By executing the instructions by the processor unit 9 at least the following steps are performed. A cleaning device 5 for applying a cleaning agent 7 to the surface 4 in the medical environment 1 is detected from a digital image taken by the camera unit 8. Furthermore, the cleaning device 5 is tracked along a cleaning trajectory 6 in the medical environment 1. From the cleaning trajectory 6 a contact probability of the cleaning device 5 with the surface 4 derived. This allows to measure the application of the cleaning agent 7 to the surface 4. All image sensors are registered with respect to each other so that e.g., a detected hand of a person 14 and cleaning devices 5 can be localized in 3D. All sensory input is fed into respective neural network to filter out the relevant information (most importantly body keypoints 15 of a person 14, keypoints of cleaning devices 16, 3D surfaces 4 of objects 3 to be cleaned). The one or more RGB sensor allow to detect relevant body parts of the person 14 and the prescribed cleaning devices 5. Typically, the system 2 will be installed such that it can monitor the patient area, but also other areas can be equipped with the system 2. The system 2 is low cost due to the use of common sensors 8, 12, 13 which can be combined in a straightforward manner. Its space demand is low so that it can be installed in any area of the medical environment. In one embodiment of the invention the at least one processor 10 is arranged to execute an artificial intelligence algorithm. The artificial intelligence algorithm being trained to detect the cleaning device 5, to track the cleaning device 5 along a cleaning trajectory 6 and to derive a contact probability of the cleaning device 5 with the surface 4 from the cleaning trajectory 6. For example, the artificial intelligence algorithm may be a trained neural network that machine learns from data of known cleanings device to detect the cleaning device 5 and to track the cleaning trajectory 6 of the cleaning device 5. The neural network can already be a trained neural network, but it can also be retrained, for example, after the camera unit has been installed in the medical environment. In another embodiment of the invention, the system 2 comprises a thermal camera 12. The thermal camera 12 also records the surfaces 4 and objects 3 to be cleaned. In doing so, the thermal camera 12 can be used to detect evaporation cooling at the surfaces 4 and objects 3 caused by the evaporation of the cleaning agent 7. This allows conclusions to be drawn about the amount of cleaning agent 7 applied to the surfaces 4 and objects 3. It may also be provided that the system 2 comprises at least one audio sensor 13. Audio sensors 13 can be easily added to the system 2 to measure friction and vibration sounds associated with cleaning activities. It is particularly useful if the sensor data of the different sensors 8, 12, 13 are synchronized with each other. In this way, the sounds can be linked to the simultaneous image positions of relevant activities, i.e., where cleaning devices 5 are used to clean a surface 4 or object 3 of interest. Also, the different sensors 8, 12, 13 can be used to assist with body keypoint 15 tracking. The data from the sensors 8, 12, 13 are combined in an overarching artificial intelligence algorithm, for example, that is trained to infer the level of cleaning or disinfection. The level of sufficient cleaning can be adapted for every cleanness category of the medical environment 1.

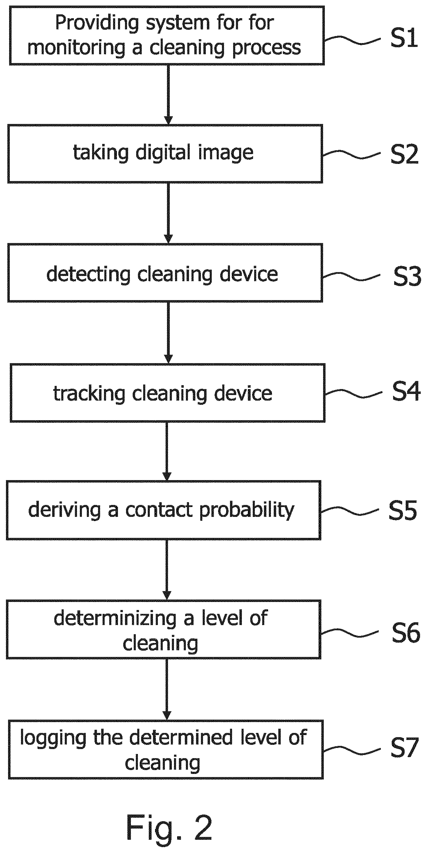

Fig. 2 depicts a flowchart of a computer-implemented method for monitoring a cleaning process of a surface 4 in a medical environment 1 according to an embodiment of the invention. The method starts in step S1 with providing a system 2 for monitoring a cleaning process of a surface 4 in a medical environment 1. The system 2 comprises at least one camera unit 8 for taking digital images of the medical environment 1, with at least one sensor for a 3D detection of the surface 4 in the medical environment 1. The system 2 further comprises at least one memory 11 for storing instructions and at least one processor 10 that executes the instructions. In step S2 a digital image is taken of the medical environment 1 by the camera unit 8. In step S3 a cleaning device 4 is detected from the digital image taken by the camera unit 8, while the cleaning device 5 is in contact with the surface 4 in the medical environment 1 to apply a cleaning agent 7 to the surface 4. In step S4 the cleaning device 5 is tracked along a cleaning trajectory 6 in the medical environment 1. In step S5 a contact probability of the cleaning device 5 with the surface 4 from the cleaning trajectory 6 is derived. In step S6 a level of cleaning of the surface 4 based on the contact probability of the cleaning device 5 with the surface 4 is determined. In step S7 the determined level of cleaning is logged. A disinfection map is thus generated and can be logged and provided as feedback and guidance to the cleaning staff. The required level of disinfection can be adapted for every cleanness category of the medical environment 1 (waiting room, changing room, imaging modality, treatment modality etc.).

In an embodiment of the invention before the computer-implemented method for monitoring a cleaning process of a surface 4 in a medical environment 1 is carried out, a computer-implemented method for monitoring a contamination of a surface 4 in a medical environment 1 is carried out and the cleaning process is carried out based on the contamination map created therein. In an embodiment of the invention the computer-implemented method for monitoring a contamination of a surface 4 in a medical environment 1 may comprise the following steps. In a first step a system 2 for monitoring a contamination of a surface 4 in a medical environment 1 is provided. The system 2 comprising at least one camera unit 8 for taking digital images of the medical environment 1, with at least one sensor for a 3D detection of the medical environment 1, at least one memory 11 storing instructions; and at least one processor 10 that executes the instructions. In a second step a digital image of the medical environment 1 is taken by the camera unit 8. In a third step from the digital image taken by the camera unit 8 at least one body part of a person 14 in the medical environment 1 is detected. In a fifth step at least one body part of the person 14 is tracked the body part of the person 14 in the medical environment 1 is tracked. In a sixth step a contact probability of the at least one body part of the person 14 with the surface 4 is derived. In a seventh step a level of contamination of the surface 4 based on the contact probability of the at least one body part of the person 14 with the surface 4 is determined. The determined level of contamination is logged in an eighth step. Finally, a contamination map of the medical environment 1 based on the logged determined level of contamination is provided. This procedure has the advantage that the cleaning process can be planned based on the previously determined contaminations. This allows the cleaning process to be designed effectively.

In an embodiment of the invention the system 2 comprises at least one thermal camera 12. The method comprises the additional steps of detecting an evaporation cooling of the cleaning agent 7 applied to the surface 4 by means of the thermal camera 12 and deriving an amount of applied cleaning agent 7 based on the detected evaporation cooling.

In an embodiment of the invention the system the system 2 comprises at least one audio sensor 13. The method comprises the following additional steps. In one additional step sounds by means of the audio sensor 13 are measured in the medical environment 1. The sounds are then linked together in a further step with a cleaning activity in the medical environment 1.

The system 2 allows more time-efficient and effective disinfection of medical environments 1 by measuring the cleaning level over the environment without interfering with other hospital workflows. A quality metric of the disinfection can be derived from the measured cleaning activity and cleaning agents 7 used. The system 2 can be installed in medical imaging and treatment environments but also in waiting areas and changing rooms. The quality metric can be tailored to the respective requirements of the environment.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope. Further, for the sake of clearness, not all elements in the drawings may have been supplied with reference signs.

**REFERENCE SYMBOL LIST**

| | |
|---|---|
| medical environment | 1 |
| system for monitoring a cleaning process | 2 |
| object | 3 |
| surface | 4 |
| cleaning device | 5 |
| cleaning trajectory | 6 |
| cleaning agent | 7 |
| camera unit | 8 |
| processor unit | 9 |
| processor | 10 |
| memory | 11 |
| thermal camera | 12 |
| audio sensor | 13 |
| person | 14 |
| body keypoints of person | 15 |
| keypoints of cleaning device | 16 |

## Claims

1. A system for monitoring a cleaning process (2) and/or a contamination of a surface (4) in a medical environment (1) the system comprising:
- at least one camera unit (8) for taking digital images of the medical environment (1), with at least one sensor for a 3D detection of the medical environment (1),
- at least one memory (11) storing instructions; and
- at least one processor (10) that executes the instructions to cause the following to be performed:
- detecting a cleaning device (5) for applying a cleaning agent (7) to the surface in the medical environment (1) from the digital image taken by the camera unit (8) and/or detecting of at least one body part of a person (14) in the medical environment (1) from the digital image taken by the camera unit (8),
- tracking the cleaning device (5) along a cleaning trajectory (6) in the medical environment (1) and/or tracking the body part of the person (14) in the medical environment (1),
- deriving a contact probability of the cleaning device (1) with the surface (4) from the cleaning trajectory (6) and/or deriving a contact probability of the at least one body part of the person (14) with the surface (4).

2. The system according to claim 1, wherein the at least one processor (10) is arranged to execute an artificial intelligence algorithm, the artificial intelligence algorithm being trained to detect the cleaning device (5), to track the cleaning device (5) along a cleaning trajectory (6) and to derive a contact probability of the cleaning device (5) with the surface (4) from the cleaning trajectory (6) and/or the artificial intelligence algorithm being trained to detect at least one body part of a person (14) in the medical environment (1) from the digital image taken by the camera unit (8), to track the body part of a person (14) and to derive a contact probability of the body part of a person (14) with the surface (4).

3. The device according to claim 2, wherein the artificial intelligence algorithm is a trained neural network that machine learns from data of known cleanings device (5) to detect the cleaning device (5) and to track the cleaning trajectory (6) of the cleaning device (5) and/or the trained neural network machine learns from data of known body parts of a person (14) to detect the body part of a person (14) and to track the body part of a person (14).

4. The system according to any preceding claim, wherein the camera unit (8) includes at least one of the following sensors, an RGB camera sensor, an ultrasonic sensor for ultrasonic ranging, a LIDAR-sensor, a time-of-flight sensor and/or a sensor for a stereo or triangulation ranging technique.

5. The system according to any preceding claim, wherein the system (2) comprises a thermal camera (12) and/or an audio sensor (13).

6. A computer-implemented method for monitoring a contamination of a surface (4) in a medical environment (1) comprising:
- providing a system for monitoring a contamination of a surface (4) in a medical environment (1) comprising:
- at least one camera unit (8) for taking digital images of the medical environment (1), with at least one sensor for a 3D detection of the medical environment (1),
- at least one memory (11) storing instructions; and
- at least one processor (10) that executes the instructions,
the method comprising the following steps:
- taking a digital image of the medical environment (1) by the camera unit (8),
- detecting of at least one body part of a person (14) in the medical environment (1) from the digital image taken by the camera unit (8),
- tracking the body part of the person (14) in the medical environment (1),
- deriving a contact probability of the at least one body part of the person (14) with the surface (4),
- determinizing a level of contamination of the surface (4) based on the contact probability of the at least one body part of the person (14) with the surface (4),
- logging the determined level of contamination,
- providing a contamination map of the medical environment (1) based on the logged determined level of contamination.

7. A computer-implemented method for monitoring a cleaning process of a surface (4) in a medical environment (1) comprising:
- providing a system for monitoring a cleaning process (2) of a surface (4) in a medical environment (1) comprising:
- at least one camera unit (8) for taking digital images of the medical environment (1), with at least one sensor for a 3D detection of the surface (4) in the medical environment (1),
- at least one memory (11) storing instructions; and
- at least one processor (10) that executes the instructions,
the method comprising the following steps:
- taking a digital image of the medical environment (1) by the camera unit (8),
- detecting a cleaning device (5) from a digital image taken by the camera unit (8), while the cleaning device (5) is in contact with the surface (4) in the medical environment (1) to apply a cleaning agent (7) to the surface (4),
- tracking the cleaning device (5) along a cleaning trajectory (6) in the medical environment (1),
- deriving a contact probability of the cleaning device (5) with the surface (4) from the cleaning trajectory (6),
- determinizing a level of cleaning of the surface (4) based on the contact probability of the cleaning device (5) with the surface (4),
- logging the determined level of cleaning.

8. The computer-implemented method according to claim 7, wherein before the method of claim 7 is carried out, the method of claim 6 is carried out and the cleaning process is carried out based on the contamination map created therein.

9. The computer-implemented method according to claims 6 to 8, wherein the method steps are at least partially performed by an artificial intelligence algorithm executed by means of the at least one processor (10).

10. The computer-implemented method according to claim 9, wherein the artificial intelligence algorithm is a trained neural network that machine learns from data of known cleanings device (5) to detect the cleaning device (5) and to track the cleaning trajectory (6) of the cleaning device (5) and/or the trained neural network machine learns from data of known body parts of a person (14) to detect the body part of a person (14) and to track the body part of a person (14).

11. The computer-implemented method according to any one of claims 7 to 10, wherein the system (2) comprises at least one thermal camera (12) and the method comprises the following additional steps:
- detecting an evaporation cooling of the cleaning agent (7) applied to the surface (4) by means of the thermal camera (12),
- deriving an amount of applied cleaning agent (7) based on the detected evaporation cooling.

12. The computer-implemented method according to any one of claims 7 to 11, wherein the system (2) comprises at least one audio sensor (13) and the method comprises the following additional steps:
- measuring of sounds by means of the audio sensor (13) in the medical environment (1),
- associating the sound with a cleaning activity in the medical environment (1).

13. The computer-implemented method according to any one of claims 7 to 12, wherein the output of different sensors (8, 12, 13) is combined in an overarching artificial intelligence algorithm, wherein a machine-learning model of the overarching artificial intelligence algorithm is trained to infer the level of achieved cleaning from the information derived by the different sensors (8, 12, 13).

14. A computer-implemented method for training an artificial intelligence algorithm to evaluate data, comprising the steps of:
- obtaining training data for an artificial intelligence algorithm, wherein the training data comprises a plurality of base training images and respective classification data for each of the base training images, and wherein the artificial intelligence algorithm is configured to receive an input image and predict classification data for the input image, wherein each image comprises data representing cleaning devices (5) and/or body parts of a person (14).

15. A computer program product comprising instructions to cause the system (2) of any one of claims 1 to 5 to execute the steps of the method of any one of claims 6 to 13.
